# EUROPEAN PATENT APPLICATION

(11) **EP 3 231 450 A1**
(43) Date of publication of application: **18.10.2017**
(21) Application number: 15866615.6
(22) Date of filing: 10.12.2015
(51) Int. Cl.: A61L 9/14, A61L 2/22, D06F 25/00, D06F 37/00, D06F 39/00

(54) **MIST GENERATOR AND WASHING MACHINE**

(30) Priority: 10.12.2014 JP 2014249578
(71) Applicant: Haier Asia Co., Ltd, Tokyo 100-0005 (JP); Qingdao Haier Washing Machine Co. Ltd., Qingdao, Shandong 266101 (CN)
(72) Inventor: FUKUI, Shinji, Tokyo 100-0005 (JP)
(74) Representative: Zacco Sweden AB
(86) International application number: PCT/CN2015/096979
(87) International publication number: WO 2016/091184

(57) **Abstract**

Provided are a mist generator (70, 70A, 70B) and a washing machine (1). The mist generator (70, 70A, 70B) can provide mist having small enough particles. The mist generator (70, 70A, 70B) includes: a housing (100, 600) having a discharge part (103, 603); a mist generation unit having a mist spraying nozzle (200, 300, 700) for spraying a mist and causing the mist sprayed from the mist spraying nozzle (200, 300, 700) to collide in the housing (100, 600) to generate a mist with particles smaller than those of the sprayed mist; and a discharging unit for generating an airflow toward the discharging opening (103, 603) in the housing (100, 600), so that in case of nonuse of a blowing fan (500, 900), the mist with smaller particles generated by the mist generation unit is discharged from the discharging opening (103, 603) along with the airflow.

## Description

### TECHNICAL FIELD

The present invention relates to a mist generator for generating mist and a washing machine having the mist generator.

### BACKGROUND

In the past, a washing machine is known to have an ozone generator and supply ozone generated by the ozone generator into a washing tank for receiving clothes so that the ozone comes into contact with the clothes to perform deodorization and sterilization etc. of the clothes (see Patent Literature 1).

In this way, an ozone removal step can be carried out in the washing machine having a deodorization/sterilization function performed through ozone, and the step promotes decomposition of ozone remaining in the washing tank through heating an interior of the washing tank by a heater at the end of a deodorization/sterilization step for deodorizing/sterilizing the clothes.

### Existing technical literature

### Patent Literature

Patent Literature 1: Japanese Patent Application Laid-Open No.2007-195896 Bulletin

### Problems to be solved by invention

As described above, when a structure for promoting decomposition of the ozone through heating performed by using a heater is adopted, relatively large electric power is liable to be consumed. In addition, the above structure is hard to apply to a case that a deodorization object and other objects are not heat-resistant. Therefore, inventors of the present application think about promoting the decomposition of the ozone by other conditions instead of heating; and results show that the decomposition of the ozone can be promoted by humidifying an interior of the washing tank after stopping supply of the ozone.

Based on such recognition, in order to promote the decomposition of the ozone, a structure for humidifying an interior of the washing tank by using an apparatus for generating mist in a washing machine can be used. In this case, it is possible to use a spray nozzle for spraying the mist as a mist generation unit.

The farther the mist sprayed from the spray nozzle is away from the spray nozzle, the smaller the particles are. However, in a relatively narrow space, that is, in the washing tank, when the structure for directly spraying the mist from the spray nozzle is adopted, a distance for making the mist become sufficiently small is difficult to be obtained between the spray nozzle and the clothes in the washing tank. Therefore, the mist is easy to contact with the clothes before becoming sufficiently small in the washing tank, and the clothes are easily wetted by the contacted mist.

### SUMMARY

A technical solution of the present invention is completed in view of the problems, and an objective of the present invention is to provide a mist generator which can supply sufficiently small mist. Further, another objective of the present invention is to provide a washing machine, and clothes in a washing tank are difficult to be wetted by the supplied mist when the mist is supplied to the washing tank having the ozone.

### Solution for solving problems

In a first aspect of the present invention, the mist generator includes: a housing having a discharge port; a mist generation unit having a spray part for spraying a mist, and causing the mist sprayed from the spray part to collide in the housing to generate the mist with particles smaller than those of the sprayed mist; and a discharge unit for generating an airflow toward the discharge port in the housing, so that the mist with small particles generated by the mist generation unit is discharged from the discharge port along with the airflow.

Through the above described structure, the mist with sufficiently small particles can be discharged from the mist generator.

In the mist generator of the present invention, the mist generation unit can include the following structure: the first spray part has a first spray part and a second spray part, and the first spray part and the second spray part are arranged in the housing in such a manner that the sprayed mists collide with each other.

Through the above described structure, since the mist is sprayed from the first spray part and the second spray part, the first spray part and the second spray part, as a whole, can spray more amount of mist, and make the generation amount of the mist with small particles much more.

In the mist generator of the present invention, the mist generation unit can include the following structure: the spray part is arranged in the housing in such a manner that the sprayed mist collides with a wall of the housing.

Through the above described structure, the mist generator for generating the mist with small particles in the housing can be realized by using one spray part.

In the mist generator of the present invention, the discharge unit can be configured to have a blower fan.

Through the above described structure, the mist with small particles generated in the housing can be discharged from the discharge port through an airflow generated by the blower fan.

In the mist generator of the present invention, when the structure including the first spray part and the second spray part is adopted, a suction port for sucking air can be arranged in the housing. In this case, the discharge unit can include the following structure: the first spray part and the second spray part are arranged in the housing in a manner of spraying the mist to each other from a suction port side toward a discharge port side.

Through the above described structure, the airflow toward the discharge port can be generated in the housing through an acting force of the mist sprayed from the first spray part and the second spray part, and the mist with small particles generated in the housing can be discharged from the discharge port by utilizing the airflow.

When the above described structure is adopted, a shielding wall is arranged downstream of the airflow closer to the discharge port than a collision position of the mist from the first spray part and the second spray part in the housing, for shielding the mist from the first spray part and the second spray part.

When the above described structure is adopted, the mist, which is sprayed from the first spray part and the second spray part and further goes to the discharge port side after collision, can be shielded by the shielding wall, thereby preventing the mist with coarse particles from being discharged from the discharge port.

In the mist generator of the present invention, a drain port is arranged in the downstream of the airflow closer to the discharge port than the collision position of the mist sprayed from the spray part at the bottom of the housing.

Through the above described structure, water other than the mist with small particles can flow to the drain port in such a manner that the water is not reverse to the airflow in the housing and can be drained from the drain port smoothly.

In a second aspect of the present invention, the washing machine includes: a washing tank for receiving clothes; an ozone generator for generating an ozone supplied to the washing tank; and a mist generator for generating a mist supplied to the washing tank with the ozone. Herein, the mist generator includes: a housing having a discharge port; a mist generation unit having a spray part for spraying a mist, and causing the mist sprayed from the spray part to collide in the housing to generate the mist with particles smaller than those of the sprayed mist; and a discharge unit for generating an airflow toward the discharge port in the housing, so that the mist with small particles generated by the mist generation unit is discharged from the discharge port along with the airflow.

Through the above described structure, the mist with sufficiently small particles can be supplied to the washing tank with the ozone, so the clothes received in the washing tank are difficult to be wetted by the supplied mist.

In the washing machine of the present invention, the mist generator includes a suction port arranged in the housing for sucking air and an ozone removal part for removing an ozone leaked outward through the suction port.

Through the above described structure, the ozone can be prevented from flowing toward outside of the washing machine through the mist generator.

### Effects of the invention

The present invention is capable of providing the mist generator which can supply the mist with sufficiently small particles. Further, the present invention is capable of providing the washing machine in which clothes in the washing tank are difficult to be wetted by the supplied mist when the mist is supplied to the washing tank with the ozone.

The effects and significance of the present invention are further clarified by description of the following embodiments. However, the following embodiments are merely examples of the present invention, and the present invention is not limited to contents recorded in the following embodiments.

### BRIEF DESCRIPTION OF DRAWINGS

FIG. 1 is a diagram illustrating a structure of an automatic clothes washing and drying machine according to an embodiment.
FIG. 2 is a diagram illustrating the other structure of the automatic clothes washing and drying machine according to an embodiment.
FIG. 3 is a diagram illustrating a structure of a mist generator according to an embodiment.
FIG. 4 is a diagram illustrating the other structure of the mist generator according to an embodiment.
FIG. 5 is a diagram for illustrating a mist generating action performed by a mist generator according to an embodiment.
FIG. 6 is a top view illustrating a structure of the mist generator according to a variant example 1.
FIG. 7 is an exploded perspective view illustrating a structure of the mist generator according to a variant example 2.
FIG. 8 is a diagram for illustrating a mist generating action performed by a mist generator according to a variant example 2.
FIG. 9 is a diagram illustrating the mist generator in which an arrangement position of a blower fan is changed according to a variant example 2.

### Reference numeral list:

1: automatic clothes washing and drying machine (washing machine) 60: ozone generator 24: washing and dewatering tank (washing tank) 70: mist generator 70A: mist generator 70B: mist generator 100: housing 103: discharge port 104: suction port 105: drain port 111: shielding wall 200: first spray nozzle (first spray part) 500: blower fan 600: housing 300: second spray nozzle (second spray part) 400: ozone removal filter (ozone removal part) 601b: bottom wall (wall) 603: discharge port 604: suction port 605: drain port 700: spray nozzle (spray part) 800: ozone removal filter (ozone removal part)

### DETAILED DESCRIPTION

Embodiments of the present invention are illustrated with reference to the drawings below.

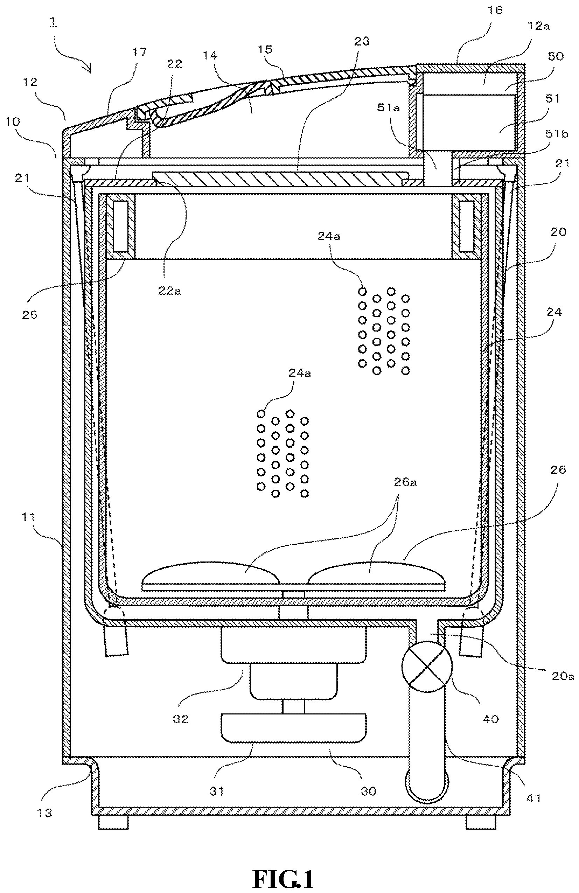

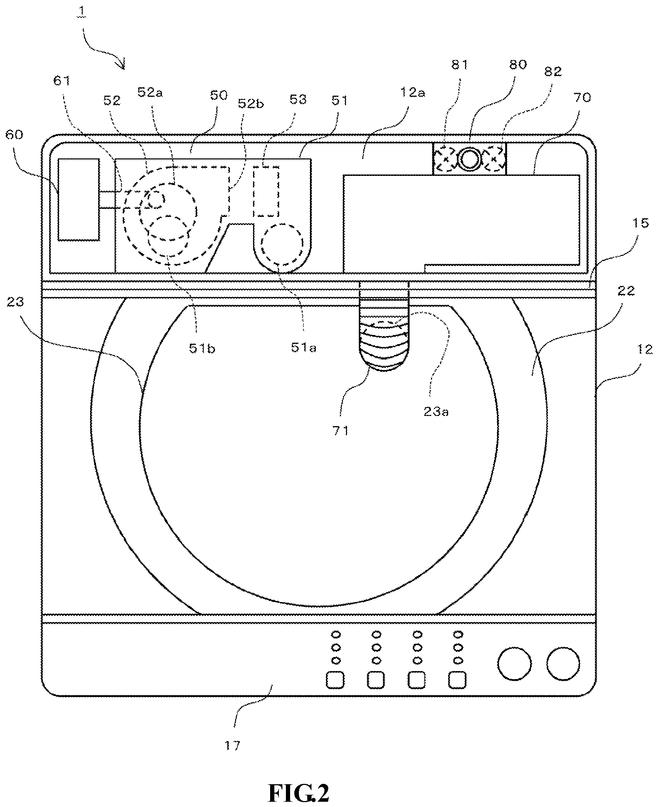

Fig. 1 and Fig. 2 are diagrams illustrating structures of an automatic clothes washing and drying machine 1. Fig. 1(a) is a side cross-sectional view of the automatic clothes washing and drying machine 1, and Fig. 2 is a top view illustrating the automatic clothes washing and drying machine 1 in a state that a rear cover 16 is removed and an upper cover 15 is opened.

The automatic clothes washing and drying machine 1 has a machine shell 10 for constituting an appearance. The machine shell 10 includes: a machine body part 11 which is of a square cylinder shape with opened upper surface and lower surface; an upper panel 12 for covering the upper surface of the machine body part 11; and a foot stand 13 for supporting the machine body part 11. A feeding port 14 of washings is formed in the upper panel 12 and covered by an upper cover 15 capable of being opened and closed freely.

In the machine shell 10, an outer tank 20 is elastically suspended and supported by four hanging rods 21 having an anti-vibration apparatus, the upper surface of the outer tank 20 is covered by an outer tank cover 22. An opening part 22a with size substantially same as that of the feeding port 14 is formed in a position of the outer tank cover 22 corresponding to the feeding port 14, and the opening part 22a is covered by the outer tank cover 22 in an opening and closing manner.

A washing and dewatering tank 24 is arranged in the outer tank 20, and a plurality of dewatering holes 24a are formed over the entire circumference of the washing and dewatering tank 24. A balancing ring 25 is arranged at an upper part of the washing and dewatering tank 24. The washing and dewatering tank 24 is equivalent to the washing tank of the present invention. A wave wheel 26 is arranged at the bottom of the outer tank 20, and a plurality of blades 26a are radially arranged on a surface of the wave wheel 26.

A driving unit 30 is arranged at the bottom of an outside of the outer tank 20. The driving unit 30 generates a torque for driving the washing and dewatering tank 24 and the wave wheel 26. The driving unit 30 includes a driving motor 31 and a transmitting mechanism part 32 which has a clutch mechanism. The torque of the driving motor 31 is only transmitted to the wave wheel 26 in a washing process and a rinsing process through switching operation performed by the clutch mechanism so that only the wave wheel 26 is rotated, and the torque of the driving motor 31 is transmitted to the wave wheel 26 and the washing and dewatering tank 24 in a dewatering process so that the wave wheel 26 and the washing and dewatering tank 24 are rotated integrally.

A drain port part 20a is formed at the bottom of the outside of the outer tank 20, and a drain valve 40 is arranged at the drain port part 20a. The drain valve 40 is connected with a drain hose 41, and when the drain valve 40 is opened, water stored in the washing and dewatering tank 24 and the outer tank 20 is discharged to the outside of the clothes washing and drying machine 1 through the drain hose 41.

A storage part 12a having an opened upper surface is arranged at a rear part of the upper panel 12. A drying unit 50, an ozone generator 60, a mist generator 70 and a water supply valve 80 are arranged within the storage part 12a, and the upper surface of the storage part 12a is covered by a rear cover 16.

A drying unit 50 includes a circulation air path 51, a circulation blower 52 and a heater 53, where the circulation air path 51 includes an exhaust cylinder 51a and a suction cylinder 51b both of which are made of elastic materials. The exhaust cylinder 51a passes through the outer tank cover 22 and has an exhaust port entering into the washing and dewatering tank 24. And the suction cylinder 51b passes through the outer tank cover 22 and has a suction port entering into a space between the washing and dewatering tank 24 and the outer tank 20. The circulation blower 52, such as a centrifugal blower, has a suction port 52a in the lower surface and a discharge port 52b in a circumferential surface, and the suction port 52a is located above the suction cylinder 51b. The circulation blower 52 circulates air between the outer tank 20 and the circulation air path 51. The heater 53 heats the circulating air in a drying process.

An ozone generator 60 is a discharge type ozone generator which generates a corona discharge, a silent discharge and so on between a pair of electrodes, and generates ozone by air passing between the pair of electrodes. The ozone generator 60 is connected to a side of a suction port 52a of the circulation blower 52 in the circulation air path 51 through an ozone supply pipe 61. When the circulation blower 52 is rotated, the ozone generated by the ozone generator 60 is sucked into the circulation blower 52 due to negative pressure at the side of the suction port 52a, and thus the sucked ozone is mixed with the circulating air and then supplied into the washing and dewatering tank 24. The ozone generator 60 can also be, for example, an ultraviolet type ozone generator, besides the discharge type ozone generator.

A mist generator 70 generates a tiny mist and supplies the generated mist into the washing and dewatering tank 24 through a supply port 23a formed in a bellows-like mist supply pipe 71 and the outer tank cover 22. The detailed structure of the mist generator 70 is described later.

A water supply valve 80 is connected with a faucet, and the water supply valve 80 is a two-way valve having a first valve 81 and a second valve 82. The first valve 81 is connected with the rear part of the outer tank 20 through a water supply hose (not shown), and in a water supply process, when the first valve 81 is opened, tap water is supplied into the outer tank 20 through the water supply hose. The second valve 82 constitutes the mist generator 70 and is connected with a spray nozzle described later, and when the second valve 82 is opened, the tap water is supplied to the spray nozzle.

An operation part 17 is arranged at a front part of the upper panel 12, and the operation section 17 is equipped with various operation buttons such as a power button, a start button and a mode selection button.

A variety of operation modes such as a washing operation, a washing and drying operation or a drying operation are performed in the automatic clothes washing and drying machine 1. The washing operation is to perform a washing process, an intermediate dewatering process, a rinsing process and a final dewatering process in sequence. The washing and drying operation is to continuously perform the operations from washing to drying, and perform a drying process after the final dewatering process. The drying operation is only to perform the drying process.

The wave wheel 26 is rotated in a rightward direction and a leftward direction in a state that water is stored in the washing and dewatering tank 24 in the washing process and the rinsing process. A water flow is generated in the washing and dewatering tank 24 through the rotation of the wave wheel 26. The washings are washed by the generated water flow and a lotion contained in the water in the washing process. The washings are rinsed by the generated water flow in the rinsing process.

The washing and dewatering tank 24 and the wave wheel 26 are integrally rotated at high speed in the intermediate dewatering process and the final dewatering process. The washings are dewatered by centrifugal force generated by the washing and dewatering tank 24.

The heated air heated by the heater 53 is circulated between the circulation air path 51 and the outer tank 20 in the drying process. In addition, the wave wheel 26 performs the right rotation and the left rotation during a given interval alternately. The washings in the washing and dewatering tank 24 are stirred by the wave wheel 26 and also are in contact with the heated air introduced into the washing and dewatering tank 24, then the heated air that absorbs a moisture from the washings by contacting the washings is discharged from the dewatering hole 24a to the space between the washing and dewatering tank 24 and the outer tank 20. Further, the heated air is cooled and dehumidified by contacting the wall of the outer tank 20 in the space, then is sucked into the circulation air path 51, next is heated by the heater 53 again and is introduced into the washing and dewatering tank 24. Thus the washings are dried.

Further, the operation in an air washing mode is also performed besides the above washing operation and the like in the automatic clothes washing and drying machine 1. The operation in the air washing mode is that ozone is supplied into the washing and dewatering tank 24 to perform a deodorization, a sterilization and so on of the clothes through the ozone.

The operation in the air washing mode includes: an ozone generation step for supplying the ozone generated by the ozone generator 60 into the washing and dewatering tank 24 to perform the deodorization, the sterilization and so on of the clothes; and an ozone decomposition step performed after the ozone generation step for decomposing and eliminating the ozone remaining in the washing and dewatering tank 24. The air washing mode is finished after the concentration of ozone in the washing and dewatering tank 24 becomes sufficiently low through the ozone decomposition step, at this time, the clothes can be taken out by unlocking the upper cover through a door lock apparatus (not shown) arranged between the upper cover 15 and the upper panel 12.

In the ozone decomposition step, the mist is supplied from the mist generator 70 into the washing and dewatering tank 24, and the interior of the washing and dewatering tank 24 is humidified by the mist, thereby promoting the decomposition of the ozone remaining in the washing and dewatering tank 24, so that the concentration of the ozone can be reduced as soon as possible.

It is believed that the reason why the concentration of the ozone is reduced by humidification is that an ozone gas is dissolved in tiny water (mist) during humidification or is decomposed through reaction. As a reaction mechanism of the ozone in the water, it is believed that the following reactions are performed.

O₃ + OH⁻→O₂⁻+ HO₂

O₃ + HO₂→2O₂ + • OH

O₃ + • OH→O₂ + HO₂

2HO₂→O3 + H₂O

HO₂ + OH→O₂ + H₂O

It should be noted that OH radicals are generated through the decomposition of ozone performed by reaction of the ozone with water based on the above reaction mechanism. Therefore, in the ozone decomposition step, the OH radicals having oxidizing power higher than that of ozone can act on the clothes, so it is expected that sebum dirt and other organic dirt attached to the clothes can be decomposed.

Since the air washing mode is mainly a mode of deodorizing and sterilizing the dried clothes, it is not expected that the clothes after deodorization, sterilization and the like are wetted by the mist introduced in order to humidify the interior of the washing and dewatering tank 24.

The automatic clothes washing and drying machine 1 in the present embodiment is characterized by the structure of the mist generator 70. As described below, the tiny mist can be supplied into the washing and dewatering tank 24 through the mist generator 70 according to the present embodiment, so the clothes in the washing and dewatering tank 24 can be hardly wetted. Hereinafter, the detailed structure of the mist generator 70 is illustrated.

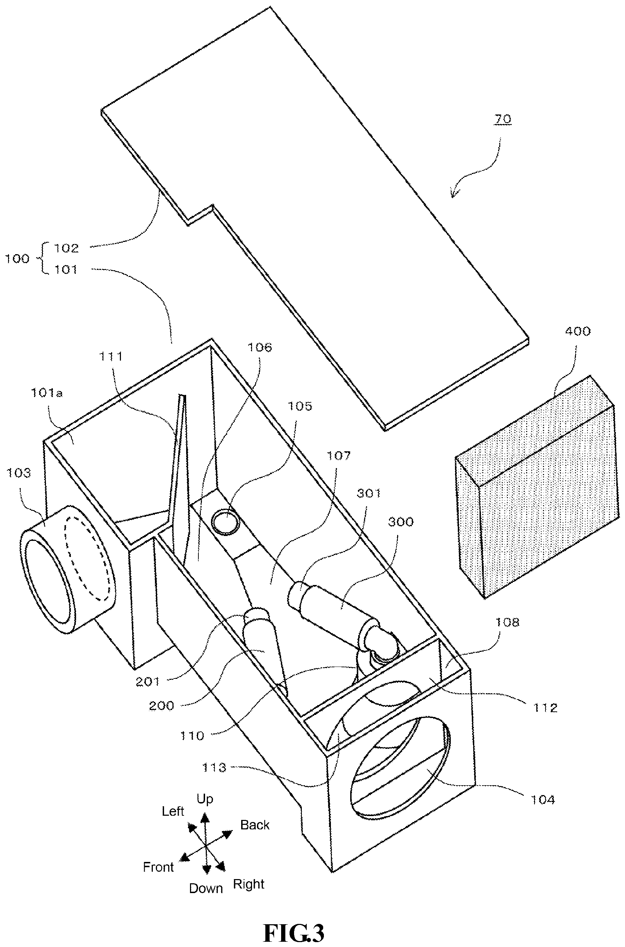

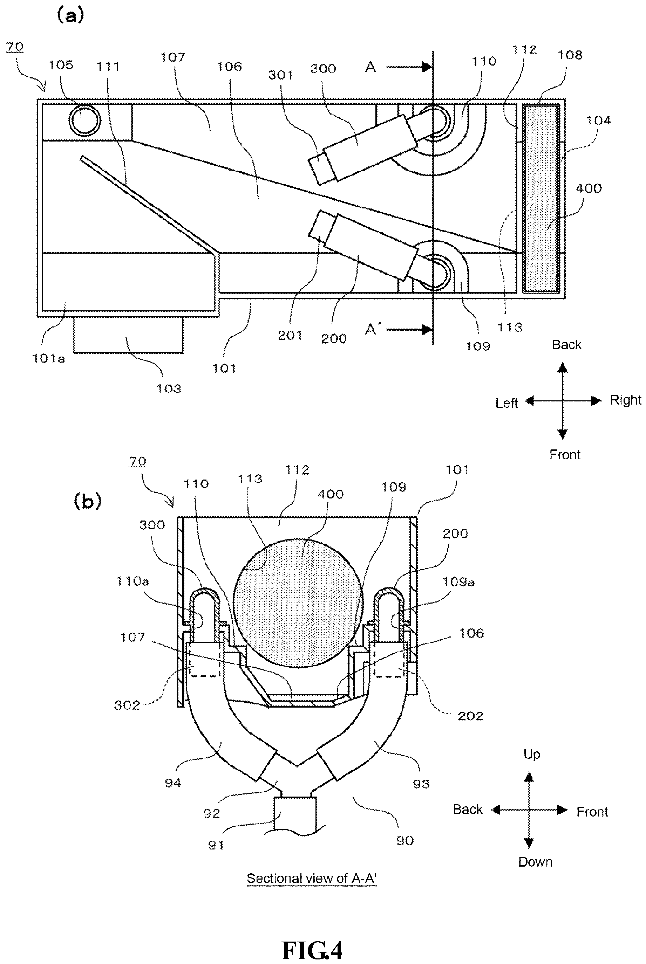

Fig. 3 and Fig. 4 are diagrams illustrating the structures of the mist generator 70. Fig. 3 is a exploded perspective viewof the mist generator 70. Fig. 4(a) is a top view of the mist generator 70 in a state that an upper surface cover 102 is removed, and Fig. 4(b) is a cross-sectional view taken along line A-A' in Fig. 4(a).

The mist generator 70 includes a housing 100, a first spray nozzle 200, a second spray nozzle 300 and an ozone removal filter 400.

The housing 100 includes a housing main body 101 and the upper surface cover 102, the housing main body 101 is formed as a substantially cubic box shape which has an opened upper surface and uses the left and right direction as the long side direction. The housing main body 101 has a protrusion part 101a slightly projecting forward at a left end part, and a cylindrical discharge port 103 is provided in the front surface of the protrusion part 101a. The discharge port 103 protrudes forward from the front surface of the storage part 12a of the upper panel 12, a mist supply pipe 71 is connected with the protruding discharge port 103. A suction port 104 for sucking air is formed in the left side of the housing main body 101.

A drain port 105 is formed in a left rear corner of the bottom surface of the housing main body 101 and is connected with the drain hose 41 by a drain hose (not shown). A first inclined surface 106 descending from the front toward the rear and a second inclined surface 107 descending from the right to the left are formed on the bottom surface of the housing main body 101 in such a manner that the water is easily converged at the drain port 105.

A filter storage part 108, a first mounting table 109, a second mounting table 110 and a shielding wall 111 are arranged within the housing main body 101.

The filter storage part 108 is formed between the left side and a partition plate 112 by arranging the partition plate 112 at a right end part of the housing main body 101, and an air vent 113 for air to pass is formed in the partition plate 112.

The first mounting table 109 is arranged at a position slightly deviating from the partition plate 112 to the left in a manner of contacting with the front surface of the housing main body 101. The second mounting table 110 is arranged at a position immediately behind the first mounting table 109 in a manner of contacting with the rear surface of the housing main body 101. The first mounting table 109 and the second mounting table 110 are raised from the bottom surface of the housing main body 101 and have cavities inside. Mounting ports 109a and 110a are formed in the upper surfaces of the first mounting table 109 and the second mounting table 110, respectively.

The shielding wall 111 is arranged in a manner of extending from the right end part of the protrusion part 101a to the left inclined rear direction. A specified gap is formed between the shielding wall 111 and the left side of the housing main body 101.

The upper surface cover 102 is a flat plate having the same shape as that of the upper surface, and is fixed to the upper surface of the housing main body 101 through a sealing member (not shown) such as a sealing gasket and the like in such a manner that the water or the air is not leaked.

The first spray nozzle 200 and the second spray nozzle 300 have the same structure and are of a substantially L shape. The first spray nozzle 200 is fixed at the mounting port 109a of the first mounting table 109 in such a manner that its ejection port part 201 is in the direction toward the left inclined rear, and when the first spray nozzle 200 is fixed to the first mounting table 109, a water inlet part 202 of the first spray nozzle 200 protrudes into the cavity inside the first mounting table 109. The second spray nozzle 300 is fixed at the mounting port 110a of the second mounting table 110 in such a manner that its ejection port part 301 is in the direction toward the left inclined front, and when the second spray nozzle 300 is fixed to the second mounting table 110, the water inlet part 302 protrudes into the cavity inside the second mounting table 110. The ejection port part 201 and the ejection port part 301 are configured to spray the mist diffused in a fan shape in a up and down direction. The first spray nozzle 200 is equivalent to the first spray part of the present invention, and the second spray nozzle 300 is equivalent to the second spray part of the present invention.

The first spray nozzle 200 and the second spray nozzle 300 are connected with a second valve 82 of the water supply valve 80 through a relay hose unit 90. As shown in Fig. 4(b), the relay hose unit 90 includes a main hose 91, a Y-shaped connector 92, a first branch hose 93 and a second branch hose 94, where the first branch hose 93 and the second branch hose 94 are connected with the main hose 91 through the Y-shaped connector 92, and the main hose 91 is connected with the second valve 82. In addition, the first branch hose 93 is connected with the water inlet part 202 of the first spray nozzle 200, and the second branch hose 94 is connected with the water inlet part 302 of the second spray nozzle 300.

The ozone removal filter 400 is arranged in the filter storage part 108. The ozone removal filter 400 is an activated carbon filter, a photocatalyst ceramic filter and the like, for removing the ozone contained in the air passing through the interior of the filter. The ozone removal filter 400 is equivalent to the ozone removal part of the present invention.

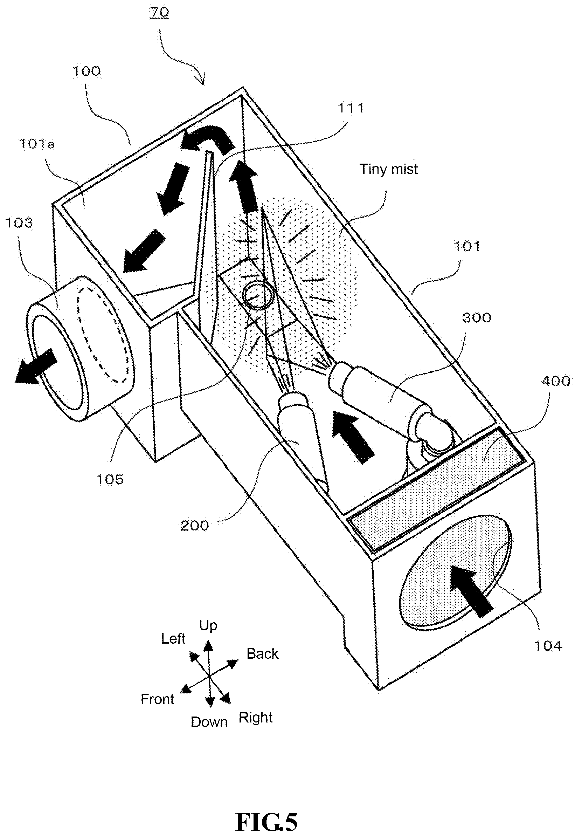

Fig. 5 is a diagram for illustrating mist generating action performed by the mist generator 70. It should be noted that, although the diagram of the upper surface cover 102 is omitted for convenience in Fig. 5, the upper surface of the housing main body 101 is closed by the upper surface cover 102 when the actual mist generating action is performed.

The water is supplied from the water supply valve 80 to the first spray nozzle 200 and the second spray nozzle 300 through the relay hose unit 90. The mist diffused in the fan shape in the up and down direction is sprayed from the first spray nozzle 200 to the left inclined rear direction, and the mist diffused in the fan shape in the up and down direction is sprayed from the second spray nozzle 300 to the left inclined fore direction. The sprayed mists collide with each other at a position closer to the present position than the shielding wall 111. The mist (hereinafter called "tiny mist") with particles smaller than those of the mist sprayed from the spray nozzles 200 and 300 is generated through the collision and is floated in the air. In the housing 100, strong mist is sprayed by the two spray nozzles 200 and 300 in a direction from the suction port 104 side to the discharge port 103 side, thus the airflow flowing from the suction port 104 side to the discharge port 103 side is generated. That is, as shown by arrows in Fig. 5, outside water is sucked into the housing 100 from the suction port 104, the sucked air goes toward the left side to penetrate through the gap between the shielding wall 111 and the left side and flow to the protrusion part 101 a, and flows outside the housing 100 from the discharge port 103. The tiny mist generated in the housing 100 goes to the discharge port 103 along with the airflow generated in the housing 100 and is discharged from the discharge port 103. The tiny mist discharged from the mist generator 70 is supplied into the washing and dewatering tank 24 through the mist supply pipe 71.

Herein, the shielding wall 111 is arranged at a downstream side closer to the air flowing in the housing 100 than a collision position of the mist sprayed by the two spray nozzles 200 and 300, and the mist further flowing to the downstream side after collision is shielded by the shielding wall 111.

In addition, the water other than the tiny mist discharged from the mist generator 70 is stored at the bottom of the housing 100 and is discharged from the drain port 105. Since the drain port 105 is arranged at the downstream closer to the airflow in the housing 100 than a mutual collision position of the sprayed mist, the water at the bottom flows to the drain port 105 in such a manner that the water is not reverse to the airflow, and is drained from the drain port 105.

As described above, in the ozone generation step, the ozone is supplied into the washing and dewatering tank 24. Since the pressure in the washing and dewatering tank 24 is higher than the external pressure, the washing and dewatering tank 24 is configured such that the ozone in the washing and dewatering tank 24 together with the air do not enter into the housing 100 through the discharge port 103 and are leaked to the outside from the suction port 104. Since the ozone removal filter 400 is arranged in a front section of the suction port 104, the ozone leaked from the suction port 104 together with the air is removed by the ozone removal filter 400.

### <Effects of the present embodiment>

As described above, according to the present embodiment, since the mist sprayed from the first spray nozzle 200 and the second spray nozzle 300 collides mutually, the mist generator 70 is configured to generate the mist with particles smaller than those of the sprayed mist in the housing and supply the generated mist with small particles into the washing and dewatering tank 24. Thus, when the interior of the washing and dewatering tank 24 is humidified to promote the decomposition of residual ozone, since the mist with sufficiently small particles can be supplied from the mist generator 70 into the washing and dewatering tank 24, the washings received in the washing and dewatering tank 24 can be difficultly wetted by the supplied mist.

In addition, according to the present embodiment, since the mist generator 70 can be configured as the structure for spraying the mist from the two spray nozzles 200 and 300, the amount of sprayed mist can be increased and the amount of generated mist with small particles can also be increased by using the two spray nozzles as a whole.

Further, according to the present embodiment, the mist generator 70 is configured to generate the airflow flowing toward the discharge port 103 in the housing 100 through the acting force of the mist sprayed from the first spray nozzle 200 and the second spray nozzle 300. Thus, the mist with small particles generated in the housing 100 can be discharged from the discharge port 103 by utilizing the airflow generated by the mist sprayed from the two spray nozzles 200 and 300.

Further, according to the present embodiment, the mist generator 70 can shield the mist, which is sprayed from the first spray nozzle 200 and the second spray nozzle 300 and then further goes to the discharge port 103 side after collision, through the shielding wall 111, thereby preventing the mist with large particles from being discharged from the discharge port 103.

Further, according to the present embodiment, the drain port 105 is arranged in the downstream closer to the airflow in the housing 100 than the mutual collision position of the sprayed mist in the mist generator 70. Thus, the water other than the mist with small particles can flow to the drain port 105 in such a manner that the water is not reverse to the airflow, and can be drained from the drain port 105 successfully.

Further, according to the present embodiment, the mist generator 70 can remove the ozone, which enters the housing 100 from the washing and dewatering tank 24 and wants to leak to the outside through the suction port 104, via the ozone removal filter 400, thereby preventing the ozone from flowing to the outside of the automatic clothes washing and drying machine 1 via the mist generator 70.

### <Variant example 1>

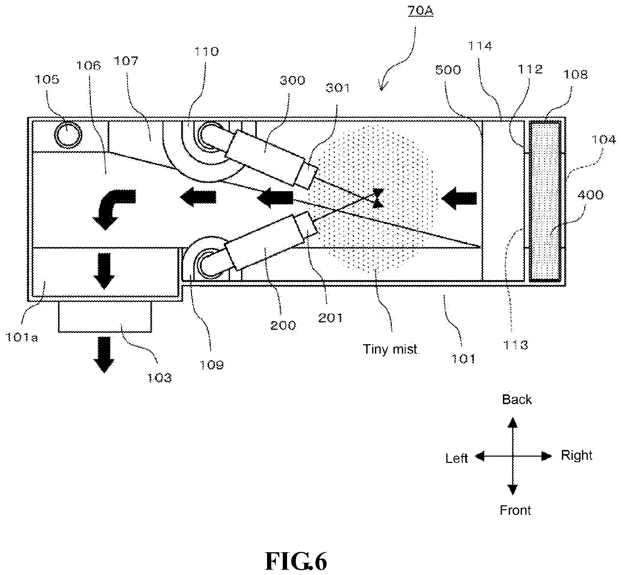

Fig. 6 is a top view illustrating the structure of a mist generator 70A according to the variant example 1.

In the mist generator 70A of the present variant example, the first mounting table 109 and the second mounting table 110 are arranged in the vicinity of the protrusion part 101a. In addition, the first spray nozzle 200 is fixed to the first mounting table 109 in such a manner that its ejection port part 201 is in the direction toward a right inclined rear, and the second spray nozzle 300 is fixed to the second mounting table 110 in such a manner that the ejection port part 301 is in the direction toward a right inclined front. Further, in the housing main body 101, an arrangement space 114 in which the blower fan 500 is arranged is on the left of the filter storage part 108, and the blower fan 500 is arranged in the arrangement space 114. The blower fan 500 is, for example, a waterproof type axial fan having the suction port in a back face of the surface opposite to the air vent 113 and having the discharge port in the front surface opposite to the air vent 113. Further, the shielding wall 111 is not arranged in the housing main body 101.

The mist diffused in the fan shape in the up and down direction is sprayed from the first spray nozzle 200 to the right inclined rear direction, and the mist diffused in the fan shape in the vertical direction is sprayed from the second spray nozzle 300 to the right inclined front direction. The sprayed mists collide with each other to generate tiny mist. As shown by the arrows in Fig. 6, the airflow flowing to the discharge port 103 is generated in the housing 100 through the action of the blower fan 500, the tiny mist goes to the discharge port 103 along with the airflow and is discharged from the discharge port 103.

Since the collided mist goes toward one side opposite to the discharge port 103, the mist with large particles is hardly leaked from the discharge port 103 even if the shielding wall is not arranged. It should be noted that the shielding wall can also be arranged when concerning about the case that the mist with large particles is returned due to the airflow and leaked from the discharge port 103 under the condition that the volume of air blown from the blower fan 500 is large and so on.

As described above, according to the present variant example, as the above embodiment, the clothes received in the washing and dewatering tank 24 are difficultly wetted by the supplied mist when the interior of the washing and dewatering tank 24 is humidified to promote the decomposition of the residual ozone.

In addition, according to the present variant example, as the above embodiment, the water other than the mist with small particles can be drained from the drain port 105 smoothly.

Further, according to the present variant example, as the above embodiment, the ozone can be prevented from flowing to the outside of the automatic clothes washing and drying machine 1 via the mist generator 70A.

Further, according to the present variant example, the mist with small particles generated in the housing 100 is discharged from the discharge port 103 well through the airflow generated by the action of the blower fan 500.

### <Variant example 2>

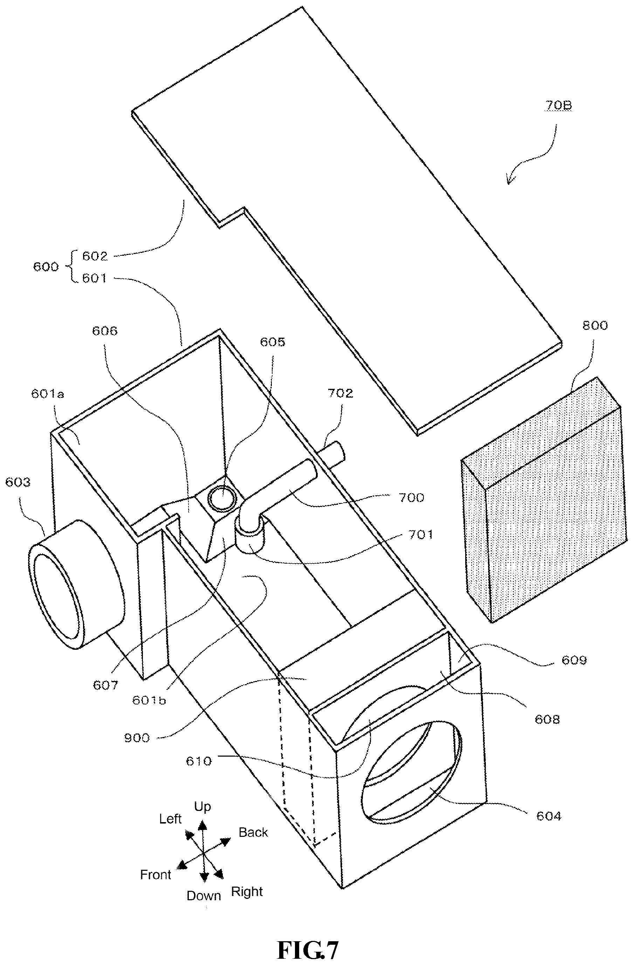

Fig. 7 is an exploded perspective view illustrating the structure of a mist generator 70B according to the variant example 2.

The mist generator 70B includes a housing 600, a spray nozzle 700, an ozone removal filter 800 and a blower fan 900.

The housing 600 includes a housing main body 601 and an upper surface cover 602. The housing main body 601 is formed as a substantially cubic box shape in which has an opened upper surface and uses the left and right direction as the long side direction. The housing main body 601 has a protrusion part 601a slightly protruding forward at the left end part, and a cylindrical discharge port 603 is provided in the front surface of the protrusion part 601a. A suction port 604 for sucking the air is formed in the left side of the housing main body 601.

A drain port 605 is formed in the left rear corner of the bottom of the housing main body 601. Further, a first inclined surface 606 descending from the front to the rear and a second inclined surface 607 descending from the right to the left are formed around the drain port 605 in such a manner that the water is easily converged at the drain port 605 at the bottom of the housing main body 601.

The right end part of the housing main body 601 is divided by a partition plate 608 to form a filter storage part 609, and an air vent 610 for the air to pass is formed in the partition plate 608.

The upper surface cover 602 is a flat plate having the same shape as that of the upper surface, and is fixed to the upper surface of the housing main body 601 via a sealing member such as a sealing gasket (not shown) and the like, in such a manner that the water and the air are not leaked.

A spray nozzle 700 has a substantially L shape and is fixed to the rear surface of the housing main body 601 in such a manner that its ejection port part 701 is downwardly arranged in the vicinity of the center of the housing main body 601. The ejection port part 701 is configured to spray the mist diffused in a fan shape in the front-rear direction. The spray nozzle 700 is equivalent to the spray part of the present invention.

When the spray nozzle 700 is fixed to the rear surface of the housing main body 601, a water inlet part 702 protrudes to the rear of the rear surface. The relay hose (not shown) is connected with the water inlet part 702. The relay hose is connected with the second valve 82 of the water supply valve 80.

The ozone removal filter 800 is arranged in the filter storage part 609. The ozone removal filter 800 may be an activated carbon filter, a photocatalyst ceramic filter and the like, for removing the ozone contained in the air passing through the interior of the filter. The ozone removal filter 800 is equivalent to the ozone removal part of the present invention.

A blower fan 900 is arranged adjacent to the filter storage part 609. The blower fan 900 is, for example, a waterproof type axial fan having the suction port in the back face of the surface opposite to the air vent 610 and a discharge port in the front surface opposite to the air vent 610.

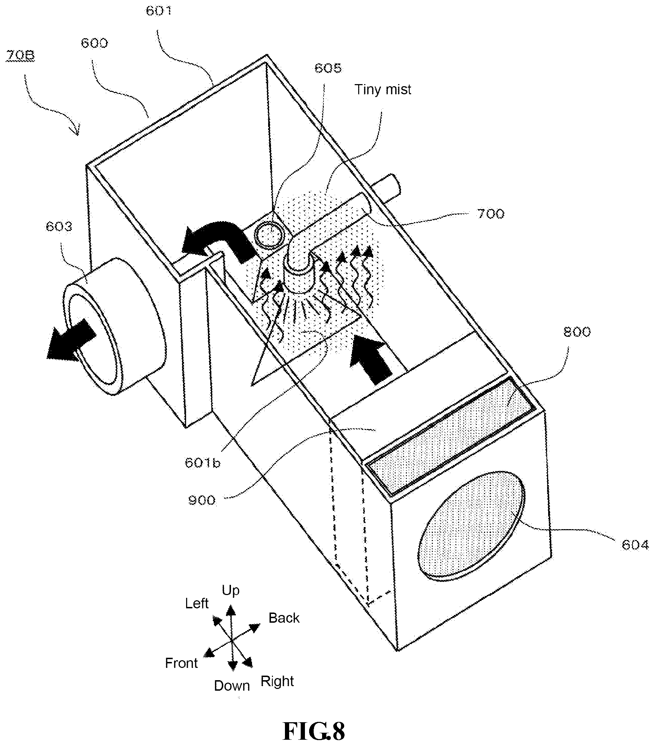

Fig. 8 is a diagram for illustrating mist generating action performed by the mist generator 70B according to the variant example 2. It should be noted that, although the diagram of the upper surface cover 602 is omitted for convenience in Fig. 8, the upper surface of the housing main body 601 is closed by the upper surface cover 602 when the actual mist generating action is performed.

When the water is supplied from the water supply valve 80 to the spray nozzle 700, the mist diffused in the fan shape in the front-rear direction is sprayed downward from the spray nozzle 700. The sprayed mists collide with a bottom wall 601b of the housing main body 601. The mist (hereinafter called "tiny mist") with particles smaller than those of the mist sprayed from the spray nozzle 700 is generated in a manner of rising from the bottom wall 601b through the collision and is floated in the air. As shown by arrows in Fig. 8, the airflow flowing from the suction port 604 to the discharge port 603 is generated in the housing 600 through action of the blower fan 900, and the tiny mist generated in the housing 600 goes to the discharge port 603 along with the airflow and is discharged from the discharge port 603.

The water other than the tiny mist discharged from the mist generator 70B is drained from the drain port 605. The drain port 605 is arranged at the downstream closer to the airflow in the housing 600 than the collision position of the sprayed mist and the bottom wall 601b of the housing main body 601. Thus, the water at the bottom flows to the drain port 605 in such a manner that the water is not reverse to the airflow and is drained from the drain port 605.

Further, in the present variant example, as the above embodiment, the ozone, which enters the housing 600 and wants to be leaked to the outside from the suction port 604, is removed by the ozone removal filter 800 in the ozone generating process.

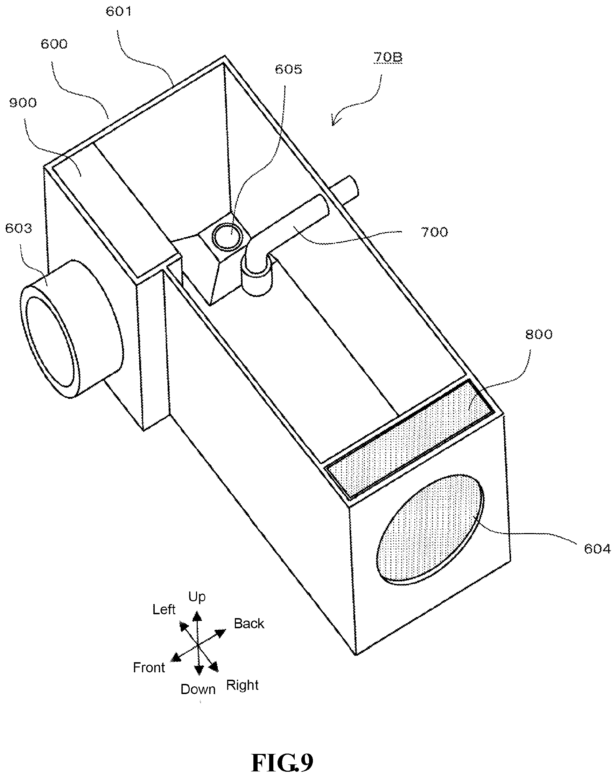

In addition, as shown in Fig. 9, the blower fan 900 can also be arranged adjacent to the discharge port 603 in the mist generator 70B according to the present variant example.

As described above, according to the present variant example, the mist generator 70B is configured to generate the mist with particles smaller than those of the sprayed mist in the housing 600 through the collision of the mist sprayed from the spray nozzle 700 and the inner wall of the housing 600, and to supply the generated mist with smaller particles into the washing and dewatering tank 24. Since the mist with sufficiently small particles is supplied into the washing and dewatering tank 24, the clothes received in the washing and dewatering tank 24 are difficult to be wetted by the supplied mist when the interior of the washing and dewatering tank 24 is humidified to promote the decomposition of the residual ozone.

In addition, according to the present variant example, it is can be achieved that the mist generator 70B is capable of generating the mist with small particles in the housing 100 by using one spray nozzle 700.

Further, according to the present variant example, as the above embodiment, the water other than the mist with small particles can be drained from the drain port 605 smoothly.

Further, according to the present variant example, as the above embodiment, the ozone can be prevented from flowing to the outside of the automatic clothes washing and drying machine 1 via the mist generator 70B.

Further, according to the present variant example, as the above variant example 1, the mist with small particles generated in the housing 600 can be well discharged from the discharge port 603 through the airflow generated by the action of the blower fan 900.

### <Other variant examples>

Embodiments of the present invention are illustrated above, but the present invention is not limited to the above embodiments and the like. In addition, the embodiments of the present invention can also be subjected to various changes other than the above changes.

For example, in the above embodiments and the variant example 1, in the mist generators 70 and 70A, the first spray nozzle 200 and the second spray nozzle 300 are arranged within the housing 100 in such a manner that their ejection port parts 201 and 301 are in the direction toward the airflow from the suction port 104 side to the discharge port 103 side. However, in the housing 100, as long as both of the mist sprayed from the first spray nozzle 200 and the mist sprayed from the second spray nozzle 300 can collide with each other, the first spray nozzle 200 and the second spray nozzle 300 may also be arranged to face any direction.

Further, in the above embodiment, the mist generator 70 is configured such that the mists sprayed from the two spray nozzles 200 and 300 collide with each other. However, the mist generator 70 is not limited to the above and can also be configured such that the mists sprayed from more than three spray nozzles collide with each other.

Further, in the variant example 2, in the mist generator 70B, the spray nozzle 700 is arranged in the housing 600 in such a manner that the sprayed mists collide with a bottom wall 601b of the housing 600. However, the spray nozzle 700 can also be arranged in the housing 600 in such a manner that the sprayed mists collide with any one of the front wall, the rear wall and the like.

Further, in the above embodiment, although the mist generator 70 mounted in the automatic clothes and washing machine 1 is exemplified, the mist generator of the present invention can be applied to other washing machines, and can also be applied to, for example, the clothes drying machine, the clothes refreshing apparatuses for fold flattening, deodorization, aromatization and the like of clothes, and other appliances.

Further, although the automatic clothes washing and drying machine 1 is exemplified in the above embodiments, the present invention can also be applied to an automatic washing machine, a drum type clothes washing and drying machine, a drum washing machine and the like.

In addition, the embodiments of the present invention can be made properly various changes within the scope of technical spirit shown in claims.

## Claims

1. A mist generator (70, 70A, 70B), comprising:
a housing (100, 600) having a discharge port (103, 603);
a mist generation unit having a spray part for spraying a mist, and causing the mist sprayed from the spray part to collide in the housing (100, 600) to generate a mist with particles smaller than those of the sprayed mist; and
a discharge unit for generating an airflow toward the discharge port (103, 603) in the housing (100, 600), so that the mist with smaller particles generated by the mist generation unit is discharged from the discharge port (103, 603) along with the airflow.

2. The mist generator (70, 70A, 70B) according to claim 1, wherein
the mist generation unit comprises the following structure: the spray part has a first spray part and a second spray part, and the first spray part and the second spray part are arranged in the housing (100, 600) in such a manner that the sprayed mists collide with each other.

3. The mist generator (70, 70A, 70B) according to claim 1, wherein
the mist generation unit comprises the following structure: the spray part is arranged in the housing (100, 600) in such a manner that the sprayed mist collides with a wall of the housing (100,600).

4. The mist generator (70, 70A, 70B) according to any one of claims 1-3, wherein
the discharge unit comprises a blower fan (500, 900).

5. The mist generator (70, 70A, 70B) according to claim 2, wherein
the housing (100, 600) is provided with a suction port (104, 604) for sucking air; and
the discharge unit comprises the following structure: the first spray part and the second spray part are arranged in the housing (100, 600) in a manner of spraying mist to each other from a suction port (104, 604) side toward a discharge port (103, 603) side.

6. The mist generator (70, 70A, 70B) according to claim 5, wherein
a shielding wall (111) is arranged at a downstream of the airflow closer to the discharge port (103, 603) than a collision position of the mist from the first spray part and the second spray part in the housing (100, 600), for shielding the mist from the first spray part and the second spray part.

7. The mist generator (70, 70A, 70B) according to any one of claims 1-6, wherein
a drain port (105, 605) is arranged downstream of an airflow closer to the discharge port (103, 603) than the collision position of the mist sprayed from the spray part at the bottom of the housing (100, 600).

8. A washing machine (1), comprising:
a washing tank for receiving clothes;
an ozone generator for generating an ozone supplied to the washing tank; and
a mist generator (70, 70A, 70B) for generating mist supplied to the washing tank with the ozone, wherein
the mist generator (70, 70A, 70B) comprises:
a housing (100, 600) having a discharge port (103, 603);
a mist generation unit having a spray part for spraying a mist, and causing the mist sprayed from the spray part to collide in the housing (100, 600) to generate the mist with particles smaller than those of the sprayed mist; and
a discharge unit for generating an airflow toward the discharge port (103, 603) in the housing (100, 600), so that the mist with small particles generated by the mist generation unit is discharged from the discharge port (103, 603) along with the airflow.

9. The washing machine (1) according to claim 8, wherein
the mist generator (70, 70A, 70B) comprises:
a suction port (104, 604) arranged in the housing (100, 600) for sucking air; and
an ozone removal part for removing the ozone leaked outward through the suction port (104,604).
